(19) Europäisches Patentamt
European Patent Office
Office européen des brevets

(11) **EP 2 381 381 A1**

(12) **DEMANDE DE BREVET EUROPEEN**

(43) Date de publication:
**26.10.2011 Bulletin 2011/43**

(51) Int Cl.:
**G06F 19/00** (2011.01)

(21) Numéro de dépôt: **11290196.2**

(22) Date de dépôt: **21.04.2011**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**

(30) Priorité: **23.04.2010 FR 1001753**

(71) Demandeur: **IFP Energies nouvelles**
**92852 Rueil-Malmaison Cedex (FR)**

(72) Inventeurs:
• **Goulon, Aurélie**
  **92160 Antony (FR)**
• **Faraj, Abdelaziz**
  **92500 Rueil-Malmaison (FR)**
• **Porcheron, Fabien**
  **69780 Mions (FR)**
• **Jacquin, Marc**
  **69002 Lyon (FR)**
• **Dartiguelongue, Cyril**
  **69360 St Symphorien d'Ozon (FR)**

(54) **Procédé pour déterminer une propriété physique ou chimique d'un composé moléculaire de structure moléculaire connue**

(57) On construit un modèle prédictif de la propriété en fonction de la structure, à partir d'une base de données constituée d'autres composés moléculaires de structures moléculaires connues. Pour ce faire : - on sélectionne dans la base un sous-ensemble de composés moléculaires représentatif de la variabilité des structures moléculaires ; pour chacun de ces composés moléculaires, on construit, au moyen d'une technique de "Graph Machines", une fonction paramétrée $g_\theta$ ayant une forme mathématique traduisant la structure moléculaire du composé ; puis, on mesure expérimentalement la propriété recherchée pour chaque composé sélectionné ; enfin, on estime les valeurs des paramètres de $g_\theta$, de façon à ce que pour chaque composé moléculaire, la fonction paramétrée $g_\theta$ donne un résultat sensiblement égal à la mesure expérimentale correspondante.

On détermine alors la propriété du composé moléculaire en construisant une fonction paramétrée pour ce composé et en utilisant les paramètres $\theta$ déterminés.

**FIG. 7**

**EP 2 381 381 A1**

**Description**

La présente invention concerne le domaine de l'analyse de composés moléculaires. En particulier, l'invention concerne un procédé pour déterminer une propriété physique ou chimique d'un composé moléculaire de structure moléculaire connue, à partir d'une base de données constituée d'autres composés moléculaires de structures moléculaires connues.

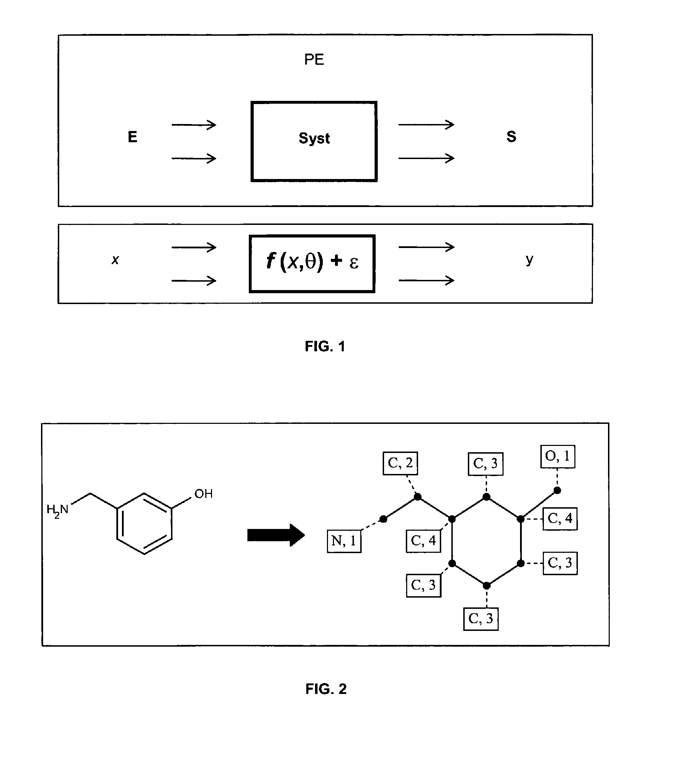

Pour déterminer une propriété physique ou chimique d'un composé moléculaire il est connu d'utiliser des techniques de modélisation d'un processus expérimental. Un processus expérimental est un système à entrées / sortie (s) - ex. réaction chimique, essai catalytique, procédé de raffinage, ...- (figure 1) dont les entrées sont des charges pouvant être constituées de mélanges de molécules. L'expérimentateur peut agir sur les entrées et les paramètres du système en fixant ou en mesurant les grandeurs caractérisant ceux-ci. La figure 1 est une représentation d'un processus expérimental (PE) en tant que système (Syst) à entrées (E) / sorties (S), ou charges / réponses, en haut de la figure, ainsi que la fonction (f (x,θ) + ε) associée au modèle de ce processus expérimental, avec les entrées notées *x* et les réponses notées *y*, en bas de la figure.

Les entrées désignent les paramètres en entrée du processus expérimental, tels que :

- la nature de la charge en entrée : formules chimiques des différentes molécules qui la constituent,

- les propriétés de la charge : viscosité,

- les familles chimiques des différents constituants de la charge,

- les concentrations des différentes molécules,

- les conditions expérimentales fixées ou mesurées dans le processus : température, pression, ...

- les paramètres fonctionnels (si les réponses sont fonctionnelles i.e. courbes ou surfaces ex. tension de vapeur, isothermes d'absorption),

Les réponses désignent les grandeurs associées aux sorties du processus expérimental, telles que :

- quantités de produits formés lors de la réaction,

- activités et propriétés physico-chimiques des produits formés,

- rendements sélectivités,

En général, les entrées et les paramètres du système sont contrôlables (ou mesurables) par l'expérimentateur alors que les réponses sont les résultats du processus et dépendent des entrées. Elles sont le résultat du système.

Dans le cadre de modélisation moléculaire, les entrées sont codées par :

- les formules chimiques des molécules constituant les charges,

- des valeurs numériques associées aux grandeurs fixées par l'utilisateur ou mesurées dans le système : paramètres du processus, propriétés des charges en entrée, concentrations des constituants, conditions expérimentales, paramètres fonctionnels, ...

- des catégories : familles chimiques des constituants, ...

Le but de la modélisation est alors de construire des modèles mathématiques, appelés *modèles de réponses* (figure 2), qui s'écrivent comme des expressions mathématiques (polynômes, réseaux de neurones, SVM, ...) des réponses en fonction des entrées. Si les grandeurs en entrée du système sont désignées par des grandeurs *x* et celles mesurées en sortie du système par des grandeurs *y*, le modèle peut s'écrire sous la forme :

$$y = f(x, \theta) + \varepsilon$$

où, *f* désigne la fonction mathématique approximant le système dont la forme dépend du modèle choisi (polynôme, ...), θ le vecteur des paramètres (inconnus) de la fonction *f* à estimer, et ε l'erreur du modèle qui modélise l'erreur expérimentale.

Ces modèles peuvent être considérés comme des approximations du processus expérimental pouvant lui être substituées. Ils peuvent servir à :

- estimer la valeur des réponses en fonction de celles des entrées,

- prédire le comportement du processus expérimental,

- optimiser le système, i.e. identifier la (les) configuration(s) de façon à améliorer le fonctionnement du processus,

- expliciter les liens pouvant exister entre ses entrées et ses sorties pour expliquer les mécanismes physico-chimiques caractéristiques du fonctionnement du processus,

**État de la technique**

La modélisation par apprentissage consiste à modéliser le comportement d'un processus à partir d'exemples de celui-ci et ainsi d'effectuer des prédictions des grandeurs étudiées. En particulier, la modélisation QSPR/QSAR (Relation Quantitative Structure-Propriété/Activité) constitue un champ de recherche important. La découverte d'une telle relation permet de prédire les propriétés physiques et chimiques et l'activité biologique de composés, de développer de nouvelles théories ou de comprendre les phénomènes observés. Elle permet également de guider la synthèse de nouvelles molécules, sans avoir à réaliser celle-ci, ou à analyser des familles entières de composés.

Les méthodes traditionnelles de modélisation sont la méthode de contribution de groupes, et les méthodes qui établissent des relations non linéaires entre les propriétés étudiées et les caractéristiques structurelles des molécules, appelées descripteurs.

Les principaux inconvénients de ces méthodes résident dans la difficulté du choix des groupes ou des descripteurs, et dans leur calcul ou leur mesure préalable.

Plus récemment, les méthodes de régression par noyaux ont été mises en oeuvre pour réaliser des prédictions à partir de structure de molécules ; elles nécessitent le choix de noyaux, qui déterminent une « distance » entre les graphes des molécules considérées, choix qui présente des difficultés importantes.

L'objet de l'invention concerne un procédé alternatif pour déterminer une propriété physique ou chimique d'un composé moléculaire de structure moléculaire connue, à partir d'une base de données constituée d'autres composés moléculaires de structures moléculaires connues. Le procédé associe une technique de "*Graph Machine*", dans laquelle on associe à chaque molécule une fonction paramétrée définie en fonction de la structure de la molécule, à une planification adaptative des mesures à réaliser. Le procédé permet ainsi de construire un modèle prédictif avec un coût expérimental le plus faible possible. On appelle coût expérimental le nombre d'expériences nécessaires à la construction du modèle. Ce nombre englobe aussi bien les expériences qui servent à estimer les paramètres du modèle que celles qui servent, d'une part, à le valider et celles qui servent, d'autre part, à tester ses capacités de prédiction.

Ce nouveau procédé présente l'avantage d'éviter le calcul des descripteurs moléculaires habituellement utilisés pour ce type de problème, puisqu'il suffit de connaître la structure en graphe de la molécule. De plus, contrairement aux méthodes traditionnelles de modélisation, les "graph machines" ne sont pas spécifiques à une propriété ou une activité donnée. Un aspect remarquable de ces fonctions paramétrées est que la même fonction peut permettre de prédire différentes propriétés, au prix seulement d'un apprentissage, et sans qu'il soit nécessaire de la reconstruire. En effet, la conversion des structures en fonction paramétrées minimise la perte d'information, contrairement à la conversion des molécules en vecteurs de données, lors de laquelle seules les informations nécessaires à la modélisation d'une propriété particulière sont conservées.

**La procédé selon l'invention**

Le procédé selon l'invention permet de déterminer une propriété physique ou chimique d'un composé moléculaire de structure moléculaire connue, à partir d'une base de données constituée d'autres composés moléculaires de structures moléculaires connues. Il comporte les étapes suivantes :

a- on sélectionne dans ladite base de données, un sous-ensemble de composés moléculaires représentatif d'une variabilité des structures moléculaires des composés de ladite base de données, au moyen d'une analyse descriptive multidimensionnelle desdites structures moléculaires ;

b- et pour chaque composé moléculaire C dudit sous-ensemble

-- on construit, au moyen d'une technique de "Graph Machines", une fonction paramétrée $g_\theta$ définie en fonction de paramètres constituant un vecteur $\theta$, et chaque fonction paramétrée $g_\theta$ ayant une forme mathématique traduisant la structure moléculaire dudit composé moléculaire $C$ ;

- on mesure expérimentalement ladite propriété dudit composé moléculaire $C$ ;

c- on estime une valeur pour chaque paramètre dudit vecteur $\theta$, de façon à ce que pour chaque composé moléculaire $C$, la fonction paramétrée $g_\theta$ donne un résultat sensiblement égale à ladite mesure expérimentale correspondante ;

d- on détermine ladite propriété physique ou chimique dudit composé moléculaire, en construisant une fonction paramétrée pour ce composé moléculaire et en utilisant lesdits paramètres $\theta$ déterminés à l'étape c.

Selon l'invention on peut construire la fonction paramétrée $g_\theta$ d'un composé *c* au moyen des étapes suivantes :

- on construit un graphe acyclique orienté représentant une structure moléculaire dudit composé *c*, et comportant un ensemble de noeuds ;

- on définit une fonction élémentaire $f_\theta$ paramétrée par ledit vecteur $\theta$ de paramètres, et associée à chaque noeud dudit graphe ;

- on définit ladite fonction paramétrée $g_\theta$ par composition de ladite fonction $f_\theta$ en fonction dudit graphe de façon à refléter ladite structure moléculaire.

On peut construire un graphe acyclique orienté, de la façon suivante pour chaque molécule d'un composé moléculaire :

- chaque atome non hydrogène est représenté par un noeud, auquel est associé une étiquette caractérisant l'atome ;
- une liaison entre deux atomes est représentée par une arête simple entre les noeuds correspondants à ces deux atomes ;

Selon l'invention, la fonction élémentaire $f_\theta$, peut être un polynôme ou un réseau de neurones. Selon un mode de réalisation, la fonction élémentaire $f_\theta$, est également fonction de propriétés liées à un atome associé au noeud auquel est associée ladite fonction élémentaire $f_\theta$, et fonction de propriétés liées à au moins une molécule dudit composé *c*. Les propriétés liées à l'atome peuvent être choisies parmi les propriétés suivantes : nature chimique, degré, stéréochimie, charge ponctuelle ; et les propriétés liées à au moins une molécule sont choisies parmi les propriétés suivantes : descripteurs moléculaires, concentrations des composés, conditions expérimentales, paramètres fonctionnels, famille chimique, mesure de types quantitatif ou qualitatif associée à l'atome.

Selon l'invention, on peut réaliser l'analyse descriptive multidimensionnelle au moyen des étapes suivantes:

- on associe à chacun des n composés moléculaires de ladite base de données, une fonction paramétrée $h_\theta$ construite au moyen d'une technique de "Graph Machines" et définie en fonction de *p* paramètres du vecteur $\theta$;

- on effectue un tirage aléatoire d'un nombre M de vecteurs $\theta$ dans l'espace $\mathbf{R}^p$ des paramètres ;

- on projette chacun des *n* composés moléculaires dans l'espace $\mathbf{R}^M$, sous forme de nuage de *n* points dont les *M* coordonnées sont les résultats de ladite fonction paramétrée $h^i_\theta$ associée à chaque composé moléculaire noté i, pour i = 1 à n ; et

- on définit une distance entre deux points à partir de laquelle on évalue une proximité entre deux composés moléculaires.

On peut réduire la dimension de l'espace $\mathbf{R}^M$ en réalisant une analyse en composantes principales, et on sélectionne lesdits composés moléculaires au moyen d'une technique de classification des premières composantes significatives issues de ladite analyse en composantes principales.

De façon avantageuse, on peut définir le vecteur de paramètres $\theta$, en définissant une fonction de coût mesurant des écarts entre lesdites mesures et lesdites valeurs prédites par ladite fonction paramétrée, et en minimisant ladite fonction de coût par modification itérative des paramètres. On peut procéder comme suit :

- on répartit lesdits composés moléculaires dudit sous-ensemble en deux bases de données : une base d'apprentissage et une base de prédiction ;

- on détermine les paramètres du vecteur θ par minimisation de ladite fonction de coût en utilisant uniquement des composés moléculaires de ladite base d'apprentissage ;

- on définit un critère traduisant un caractère prédictif de ladite fonction paramétrée, à partir de ladite base de prédiction ;

- on modifie lesdits paramètres du vecteur θ de façon itérative jusqu'à ce que ledit critère soit conforme à un seuil donné.

Selon un mode de réalisation, on peut sélectionner d'autres composés moléculaires si ledit critère est conforme à un seuil donné.

Chaque composé moléculaire peut comporter plusieurs molécules. On construit alors un graphe acyclique orienté pour ledit composé moléculaire par assemblage de graphes acycliques orientés construits pour chacune desdites molécules.

Les composés moléculaires peuvent être des hydrocarbures, et la propriété physique ou chimique dudit composé moléculaire peut être l'indice de cétane desdits hydrocarbures.

D'autres caractéristiques et avantages du procédé selon l'invention, apparaîtront à la lecture de la description ci-après d'exemples non limitatifs de réalisations, en se référant aux figures annexées et décrites ci-après.

**Présentation succincte des figures**

- La figure 1 est une représentation d'un processus expérimental en tant que système à entrées / sorties (en haut), ainsi que la fonction associée au modèle de ce processus expérimental (en bas).

- La figure 2 montre la représentation d'une molécule (à gauche) par un graphe étiqueté (à droite).

- La figure 3 représente la conversion du graphe cyclique de la figure 2 en arborescence.

- La figure 4 représente une molécule (à gauche), l'arborescence associée (à droite), et la Graph Machine ($g_\theta$ en bas) construite à partir de cette arborescence.

- La figure 5 représente un exemple de codage d'un mélange de deux molécules par un graphe unique.

- La figure 6 illustre un exemple de structure de la fonction élémentaire lorsqu'il s'agit d'un réseau de neurones.

- La figure 7 illustre la boucle de planification adaptative.

- La figure 8 montre des courbes des distributions du critère de qualité du modèle construit à chaque itération de la boucle de planification adaptative.

- La figure 9 représente la structure de la fonction élémentaire choisie pour l'analyse factorielle.

- La figure 10 représente les images des 146 hydrocarbures dans le plan des deux composantes principales (Dim1 et Dim2) obtenues par ACP.

- La figure 11 représente les exemples de la base d'apprentissage et de la base de prédiction, dans le plan des deux premières composantes principales de l'ACP.

- La figure 12, représente l'indice de cétane prédit (ICp) en fonction de l'indice de cétane expérimental (ICex).

- La figure 13 illustre la variation de l'écart-type des prédictions ($\sigma_{moy}$) en fonction des itérations (IT).

- La figure 14 illustre la variation de l'erreur quadratique moyenne de prédiction (RMSE$_{moy}$) en fonction des itérations (IT).

- La figure 15 représente les valeurs estimées par le modèle sur l'ensemble des bases de prédiction et d'apprentissage, à la dixième itération.

## Description détaillée du procédé

Le procédé selon l'invention permet de déterminer une propriété physique ou chimique d'un composé moléculaire, connaissant sa structure moléculaire, et disposant d'une base de données constituée d'autres composés moléculaires de structures moléculaires connues.

Le procédé comporte les étapes suivantes :

1. Constitution d'une base de données de mélanges moléculaires

2. Sélection d'hydrocarbures représentatifs, par analyse descriptive multidimensionnelle

3. Détermination expérimentale des propriétés des molécules sélectionnées

4. Construction du modèle de réponse

5. Utilisation du modèle de réponse

Selon un exemple de réalisation, utilisé pour décrire l'invention, les composés moléculaires sont des hydrocarbures. La propriété physique ou chimique est l'indice de cétane d'un hydrocarbure.

### 1. Constitution d'une base de données d'hydrocarbures

Cette étape est facultative si l'on dispose déjà d'une telle base de données.

Cette base de données contient pour chaque hydrocarbure, leur structure moléculaire, c'est-à-dire la nature des atomes qui constituent les molécules, l'isomérie, la topologie des atomes.

Cette base peut également contenir, pour tous ou certains hydrocarbures :

- des propriétés intrinsèques à la (aux) molécule(s), telles que les descripteurs moléculaires;

- des propriétés caractéristiques de l'hydrocarbure : viscosité, température de fusion, ... ;

- des variables externes :

  ○ paramètres des conditions expérimentales (P, T, etc.),

  ○ paramètres fonctionnels si les propriétés à modéliser sont des courbes ou des surfaces dépendant de grandeurs (paramètres fonctionnels) qui varient ou que l'on fait varier : ex. le temps de réaction, la température, etc

  ○ variables catégorielles, ...

- les réponses expérimentales (indice de cétane).

Selon l'invention, un composé moléculaire au sein de cette base de données, peut être constitué d'une unique molécule.

### 2. Sélection d'hydrocarbures représentatifs, par analyse descriptive multidimensionnelle

Pour construire un modèle, capable de prédire l'indice de cétane d'un hydrocarbure, connaissant sa structure, il est nécessaire de posséder un ensemble d'hydrocarbures pour lesquels l'indice de cétane est connu, de manière à calibrer ce modèle.

Ces indices de cétane peuvent être déterminés par des expériences classiques de laboratoire.

Cependant, ces expériences sont longues et couteuses. Il est donc important, voir indispensable, de réduire au maximum ces expériences, tout en conservant un nombre suffisant d'hydrocarbure pour lesquels l'indice de cétane est connu, de façon à construire un modèle prédictif.

C'est pourquoi, selon l'invention, on sélectionne dans la base de données, un sous-ensemble de composés moléculaires représentatif de la variabilité des structures moléculaires des composés de cette base de données. Pour ce faire, on pratique une analyse descriptive multidimensionnelle des hydrocarbures de la base de données. Cette analyse peut être réalisée de deux manières :

> a. Analyse des relations entre les hydrocarbures constituant cette base de données.
> Les résultats de cette analyse peuvent être représentés par
>
> > i. un arbre de classification donnant des classes qui traduisent les ressemblances entre ces hydrocarbures du point de vue de leurs structures, caractéristiques physico-chimiques, conditions expérimentales, etc.
> >
> > ii. des projections d'hydrocarbures en tant que points dans des espaces synthétiques, où les distances entre ces points rendent compte des similitudes pouvant exister entre ces hydrocarbures.
>
> b. Analyse des liens pouvant exister entre les variables explicatives, les réponses (si celles-ci sont disponibles) et les classes d'hydrocarbures.

Selon un mode de réalisation, les projections d'hydrocarbures en tant que points dans des espaces synthétiques, au cours de l'analyse des relations entre les hydrocarbures, est réalisée au moyen de la technique des "*Graph Machine*". Cette analyse comporte alors les étapes suivantes:

> i. on associe à chaque hydrocarbure de la base de données une fonction paramétrée
>
> ii. on réalise une projection de chaque hydrocarbure en tant que points
>
> iii. on définit une distance entre deux points
>
> iv. on sélectionne les hydrocarbures en fonction des distances

i. Association à chaque hydrocarbure d'une fonction paramétrée

On associe à chacun des $n$ composés moléculaires de la base de données, une fonction paramétrée qui reproduit la structure du composé moléculaire. Elle est définie en fonction de $p$ paramètres d'un vecteur θ, et elle est construite selon une technique connue des spécialistes : les *"Graph Machines"*. Cette technique est entièrement décrite dans les documents suivants :

- Goulon-Sigwalt-Abram, A. Duprat and G. Dreyfus: "From Hopfield nets to recursive networks to graph machines: Numerical machine learning for structured data", Theoretical Computer Science, Volume 344, Issues 2-3, 17 November 2005, pages 298-334.

- Goulon, T. Picot, A. Duprat, G. Dreyfus: "Predicting activities without computing descriptors: graph machines for QSAR", SAR and QSAR in Environmental Research, Volume 18, Issue 1 & 2 January 2007, pages 141- 153.

- Goulon, A; Duprat, G; Dreyfus: "Graph Machines and Their Applications to Computer-Aided Drug Design: A New Approach to Learning from Structured Data", Lecture Notes in Computer Science, Volume 4135/2006, pages 1-19 *(Invited paper)*.

- Goulon-Sigwalt-Abram, A. Duprat, G. Dreyfus: "Learning numbers from graphs", Applied Statistical Modeling and Data Analysis (2005)

Selon cette technique, chaque composé moléculaire est dans un premier temps représenté par un graphe non orienté, cyclique ou acyclique, qui tient compte des liaisons chimiques, de la nature des atomes ou encore de la stéréochimie du composé initial. Puis, dans un second temps, ce graphe est transformé en graphe acyclique orienté. Enfin, on construit à partir de ce graphe orienté une fonction mathématique, appelée "Graph Machine", dont la structure reproduit celle du graphe orienté. Selon un mode de réalisation, on construit un graphe acyclique orienté, de la façon suivante pour chaque molécule d'un composé moléculaire :

- des atomes d'hydrogène ne sont pas directement pris en compte ;
- chaque atome non hydrogène est représenté par un noeud, auquel est associé une étiquette caractérisant l'atome ;
- une liaison entre deux atomes est représentée par une arête simple entre les noeuds correspondants à ces deux atomes ;

A chaque graphe acyclique orienté (ou arborescence) est associée une fonction mathématique, appelée *Graph Machine,* dont la structure reproduit celle de l'arborescence. On définit une fonction élémentaire $f_\theta$, paramétrée par un vecteur de paramètres $\theta$. Cette fonction peut être une fonction linéaire, un polynôme, ou un réseau de neurones par exemple. A chaque noeud de l'arborescence est associée cette fonction $f_\theta$, appelée pour cette raison *fonction de noeud,* où $\theta$ est identique pour tous les noeuds.

Pour chaque graphe $G_i$, on construit alors une fonction $g_\theta^i$ par composition des fonctions $f_\theta$, de façon à refléter la structure du graphe : si $s_a$ et $s_b$ sont deux sommets du graphe, tels que $a$ est parent de $b$ (i.e. un arc part de $s_a$ et arrive en $s_b$), alors le résultat de la fonction associée au noeud $s_b$ est argument de celle associée au noeud $s_a$.

La fonction de noeud paramétrée $f_\theta$ associée au noeud z est donc de la forme:

$$f_\theta(z) = f(u,v)$$

où :

- u est un vecteur dont les composantes sont égales aux arguments de sorties des fonctions associées aux noeuds parents du noeud z en question

- v est un vecteur optionnel dont les composantes fournissent l'information localisée au noeud : ce sont les étiquettes du noeud pouvant être une valeur qualitative (comme la nature du noeud) ou quantitative (comme les descripteurs associés à la molécule).

La fonction élémentaire $f_\theta$ peut également être fonction de propriétés liées à un atome associé au noeud auquel elle est associée: nature chimique, degré, stéréochimie, charge ponctuelle, ... La fonction élémentaire $f_\theta$ peut également être fonction de propriétés liées à au moins une molécule du composé moléculaire : descripteurs moléculaires, concentrations des composés, conditions expérimentales, paramètres fonctionnels, famille chimique, mesure de types quantitatif ou qualitatif associée à l'atome.

Finalement, la sortie de la fonction $g_\theta^i$ est la sortie de la fonction associée au noeud racine.

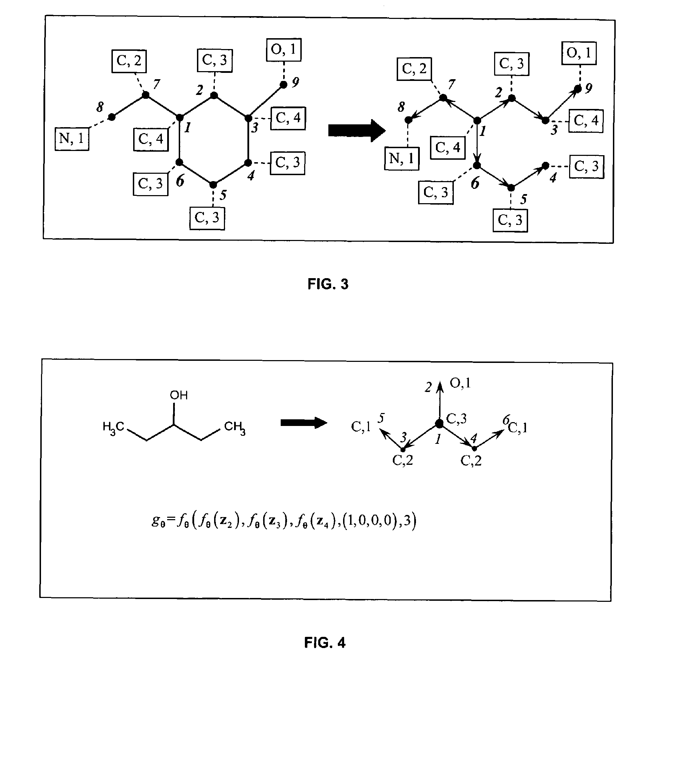

La figure 4 représente ainsi une molécule, l'arborescence associée, et la fonction paramétrée (*Graph Machine*) construite à partir de cette arborescence. La sortie de cette fonction paramétrée est celle de la fonction associée au noeud 1. Cette fonction prend pour argument les sorties des fonctions associées aux noeuds 2, 3 et 4, qui sont les enfants du noeud 1, ainsi que les informations portées par l'étiquette du noeud 1, c'est-à-dire la nature de l'atome correspondant et son degré :

$$g_\theta = f_\theta\left(\underbrace{f_\theta(z_2), f_\theta(z_3), f_\theta(z_4)}_{\substack{\text{Sorties des fonctions des} \\ \text{nœuds 2, 3 et 4 enfants du} \\ \text{noeud 1}}}, \underbrace{(1,0,0,0)}_{\text{atome : C}}, \overset{\nearrow}{3}\right) \quad \text{Degré 3}$$

Ainsi, à une base d'hydrocarbures est associé un ensemble de fonctions paramétrées, appelées *"Graph Machines",* partageant le même jeu de paramètres.

Une description détaillée de la construction par *"Graph Machines"* est fournie dans l'annexe 1.

ii. Projection de chaque hydrocarbure en tant que points

Soit $\Omega$ l'ensemble des hydrocarbures candidats à l'expérimentation (étape 3). Le codage de ceux-ci par *"Graph Machines"* permet d'associer à tout hydrocarbure $\omega$ de $\Omega$ une fonction paramétrée $g_\theta^\omega$. Ceci induit la définition de deux fonctions, notées respectivement $g_\theta$ et $g^\omega$.

La fonction $g_\theta$ est définie sur l'ensemble $\Omega$ des hydrocarbures de la base de données et à valeur dans $\mathbf{R}$ :

$$g_\theta : \Omega \quad \rightarrow \quad \mathbf{R}$$

$$\omega \quad \mapsto \quad g_\theta^\omega$$

La fonction $g^\omega$ est définie sur l'ensemble $\mathbf{R}^p$ des paramètres et à valeurs dans $\mathbf{R}$ :

$$g^\omega : \mathbf{R}^p \quad \rightarrow \quad \mathbf{R}$$

$$\theta \quad \mapsto \quad g_\theta^\omega$$

En faisant un tirage aléatoire par la méthode de Monte Carlo d'un nombre M de paramètres $\theta^m$ dans l'espace $\mathbf{R}^p$, nous pouvons associer à tout hydrocarbure $\omega$ de $\Omega$ les M valeurs $g_{\theta^m}^\omega$ qui définissent le point ( $g_{\theta^1}^\omega$ , $g_{\theta^2}^\omega$ , ..., $g_{\theta^M}^\omega$ de dimension M.

Ainsi, tout hydrocarbure $\omega$ peut-être représenté par un point dans l'espace $\mathbf{R}^M$ de sorte que l'ensemble des hydrocarbures candidats se trouvent représentés par un nuage de N points de $\mathbf{R}^M$.

iii. Définition d'une distance entre deux points

L'espace $\mathbf{R}^M$ étant muni d'une distance $\mathbf{d}$, il est possible d'évaluer la proximité entre deux hydrocarbures i et i' à l'aide de cette distance par :

$$\mathbf{d}^2(i,i') = \sum_{m=1}^{q} \left( g_{\theta^m}^i - g_{\theta^m}^{i'} \right)^2$$

iv. Sélection d'hydrocarbures en fonction des distances

On sélectionne un sous-ensemble d'hydrocarbures, en choisissant des hydrocarbures éloignés les uns des autres, indiquant des différences de structures importantes, et donc, a priori, des indices de cétane bien différents.

Pour faciliter la sélection, on réalise de préférence une analyse en composantes principales suivie d'une classification, comme décrit ci-après.

Le codage par *"Graph Machines"* de ces charges et le tirage par Monte Carlo des M vecteurs de paramètres dans l'espace $\mathbf{R}^p$ permet de créer un tableau de dimension N x M contenant en lignes les N hydrocarbures et en colonnes les valeurs $g_{\theta^m}^i$ associées aux hydrocarbures pour les M paramètres.

Il est possible de réduire le nombre M de dimensions de ce tableau en lui appliquant une analyse en composantes principales (ACP). Soit q le nombre de composantes retenues suite à cette ACP et $c^1$, $c^2$, ..., $c^q$ les composantes principales en question, la distance entre deux hydrocarbures i et i' quelconques peut s'écrire dans l'espace de ces composantes :

$$d^2(i,i') = \sum_{m=1}^{q} \left( c_i^m - c_{i'}^m \right)^2$$

On applique ensuite une méthode de classification (CLASS) automatique à l'ensemble des hydrocarbures de façon à les organiser en un nombre K de classes homogènes et bien séparées les unes des autres.
On sélectionne ensuite au moins un hydrocarbure par classe.

3. Détermination expérimentale des propriétés des molécules sélectionnées

Au cours de cette étape on détermine expérimentalement l'indice de cétane des hydrocarbures sélectionnés à l'étape 2, si cet indice n'est pas déjà présent dans la base de données.

4. Construction du modèle de réponses

A partir des hydrocarbures sélectionnés, pour lesquels on connaît l'indice de cétane et on dispose d'un ensemble de fonctions composées (*"Graph Machines"*) partageant le même jeu de paramètres, la modélisation de l'indice de cétane consiste alors à déterminer ces paramètres par apprentissage. Cette approche a l'avantage de dispenser de calculer les descripteurs moléculaires habituellement utilisés pour ce type de problème, puisqu'il suffit de connaître la structure en graphe de la molécule.
Cette étape consiste ainsi à estimer une valeur pour chaque paramètre du vecteur θ, de façon à ce que pour chaque composé moléculaire sélectionné, la fonction paramétrée $g_\theta$ donne un résultat sensiblement égale à la mesure expérimentale correspondante.

Construction du modèle par apprentissage :

L'apprentissage consiste à estimer le vecteur de paramètres θ à partir d'un ensemble d'exemples formant la base d'apprentissage.
Lors de l'apprentissage des fonctions paramétrées, la base d'apprentissage est constituée de *N* couples fonction paramétrée- sortie $\left\{ \left( g_\theta^i, y_i \right), i = 1, ..., N \right\}$.

Il est alors possible de définir une fonction de coût similaire à la fonction de coût des moindres carrés traditionnelle. Cette fonction mesure les écarts entre les observations et les valeurs prédites par le modèle :

$$J(\theta) = \sum_{i=1}^{N} \left( y_i - g_\theta^i \right)^2$$

La minimisation de la fonction de coût s'effectue de la même manière que lors d'un apprentissage classique, en modifiant les paramètres de façon itérative en fonction de son gradient.
Les algorithmes traditionnels de descente du gradient, tels que Levenberg-Marquardt, BFGS ou le gradient conjugué, peuvent alors être mis en oeuvre pour minimiser la fonction de coût, et trouver le vecteur de paramètres θ correspondant.

Sélection du modèle :

La modélisation vise à fournir un modèle qui soit non seulement ajusté aux données d'apprentissage, mais aussi capable de prédire la valeur de la sortie sur de nouveaux exemples, c'est-à-dire de généraliser. L'erreur du modèle sur les données d'apprentissage, $E_{train}$, peut être évaluée par l'erreur quadratique moyenne en apprentissage, appelée également EQMA :

$$E_{train} = \sqrt{\frac{1}{N_{train}} \sum_{i=1}^{N_{train}} (R_i)^2}$$

où $N_{train}$ est le nombre d'exemples servant à établir le modèle.

L'erreur sur la base d'apprentissage seule n'est pas un bon indicateur de la qualité du modèle ; un bon modèle doit être capable de rendre compte de la relation déterministe entre les variables et la grandeur modélisée, sans s'ajuster au bruit des données d'apprentissage. Il convient donc de trouver comment estimer cette capacité.

La méthode la plus commune consiste à répartir hydrocarbures du sous-ensemble en deux bases de données : une base d'apprentissage et une base de prédiction. Les paramètres du modèle sont ajustés sur la base d'apprentissage, et l'erreur de généralisation est évaluée sur la base de validation :

-   on détermine les paramètres du vecteur $\theta$ par minimisation de la fonction de coût en utilisant uniquement les composés moléculaires de la base d'apprentissage ;
-   on définit un critère traduisant un caractère prédictif de la fonction paramétrée, à partir de la base de prédiction ;
-   on modifie les paramètres du vecteur $\theta$ de façon itérative jusqu'à ce que le critère soit conforme à un seuil donné.

L'erreur en validation (*VAL*) est définie de façon analogue au coût d'apprentissage :

$$E_{valid} = \sqrt{\frac{1}{N_{valid}} \sum_{i=1}^{N_{valid}} (R_i)^2}$$

où $N_{valid}$ est le nombre d'exemples dans la base de validation.

Pour des modèles de complexité donnée, la méthode de sélection consiste à effectuer plusieurs apprentissages à partir de différentes initialisations des paramètres. Il faut alors choisir, parmi les modèles obtenus, celui qui fournit la plus faible erreur en validation.

*La validation croisée (VALC)*

L'ensemble des N exemples disponibles est cette fois-ci divisé en K sous-ensembles disjoints. On construit alors une base d'apprentissage à partir de K-1 de ces sous-ensembles. Le modèle est ajusté sur cette base, puis l'erreur de prédiction $R_i$ est calculée pour chaque exemple du sous-ensemble restant. Cette étape est réalisée K fois, en choisissant un sous-ensemble de validation différent à chaque itération. Ainsi, chaque exemple se trouve une fois et une seule dans une base de validation.

La performance en généralisation de la famille de modèles est ensuite estimée en calculant le score de validation croisée :

$$\sqrt{\frac{1}{N} \sum_{i=1}^{N} (R_i)^2}$$

*Méthode du leave-one-out*

Le cas particulier où K=N (N est le nombre d'exemples disponibles) est appelé *leave-one-out* : à chaque itération, un exemple i est extrait de l'ensemble d'apprentissage. Une série d'apprentissage est réalisée à l'aide des N-1 exemples restants, et l'erreur de prédiction sur l'exemple *i* est calculée pour chacun des modèles obtenus. On retient l'erreur la plus faible, notée $R_i^{-i}$. Le score de leave-one-out est alors défini par :

$$E_{loo} = \sqrt{\frac{1}{N} \sum_{i=1}^{N} \left(R_i^{-i}\right)^2}$$

Critères de qualité d'un modèle GM :

$Z_{cand} = \{x_i, i = 1, ..., N\}$ : ensemble de N individus candidats à l'expérimentation (les valeurs de la réponse $y$ ne sont pas disponibles).

$Z_{train} = \{(x_i, y_i), i = 1, ..., n_{train}\}$ : ensemble de $n_{train}$ expériences qui servent en tant que données d'apprentissage.

$Z_{valid}$ : ensemble des données de validation servant à "ajuster" les paramètres du modèle.

$Z_{pred}$ : ensemble de $n_{pred}$ expériences de prédiction servant à tester le modèle.

$Z_{train}$, $Z_{valid}$ et $Z_{pred}$ sont des sous-ensembles sélectionnés dans $Z_{cand}$ pour lesquels les valeurs de la réponse $y$ sont disponibles.

On construit L modèles $\hat{y}^\ell = g_{\hat{\theta}^\ell}$ à partir de l'ensemble d'apprentissage $Z_{train}$. Chaque modèle est obtenu par une initialisation différente pour l'estimation des paramètres $\theta$ dans l'algorithme d'optimisation de GM.

Chaque modèle $g_{\hat{\theta}^\ell}$ est ensuite appliqué aux individus appartenant successivement aux données de calibration $Z_{train}$, de prédiction $Z_{pred}$ et candidats $Z_{cand}$.

On note : $\hat{y}_i^\ell = g_{\hat{\theta}^\ell}(x_i)$ si $x_i \in Z_{train}$ (valeur prédite pour un individu i ayant servi à construire le modèle)

$\hat{y}_{(-i)}^\ell = g_{\hat{\theta}^\ell}(x_i)$ si $x_i \in Z_{pred}$ (valeur prédite pour un individu i n'ayant pas servi à construire le modèle)

On définit les critères suivants

$$RMSEP_{pred}^{\ell}{}^2 = \frac{1}{n_{pred}} \sum_{x_i \in Z_{pred}} \left(\hat{y}_i^\ell - y_i\right)^2 \quad \textbf{(1)}$$

$$R_{pred}^{\ell}{}^2 = cor^2(\hat{y}^\ell, y) \qquad\qquad \textbf{(2)}$$

avec $\hat{y}^\ell = (\hat{y}_i^\ell)^T$ et $y = (y_i)^T$ pour i=1, $n_{pred}$.

L'ensemble $\{RMSEP_{pred}^\ell, \ell = 1, L\}$ représente la distribution empirique de la racine de l'erreur quadratique moyenne de validation croisée. Calculée pour les individus n'ayant pas servi dans la construction du modèle, $RMSEP_{pred}^\ell$ est un indicateur de l'erreur de généralisation du modèle. Ses valeurs sont d'autant plus proches de 0 que le modèle a un bon pouvoir de généralisation. 2

L'ensemble $\{R_{pred}^{\ell}{}^2, \ell = 1, L\}$ représente la distribution empirique du $R^2$ calculé par validation croisée.

Les valeurs de $R_{pred}^{\ell}{}^2$ sont d'autant plus élevées (proches de 1) que le modèle a un bon pouvoir de prédiction.

La deuxième catégorie d'indicateurs est constituée de ceux portant sur la qualité de prédiction pour un individu i dans $Z_{pred}$, erreur de prédiction $e_{(-i)}^\ell$ :

$$e_{(-i)}^{\ell} = \hat{y}_{(-i)}^{\ell} - y_i \ \text{pour} \ \ell = 1, L \quad (3)$$

La variance de prédiction $\sigma_i^2$ (ou écart-type de $\sigma_i$ prédiction) définie ci-dessous peut être calculé en n'importe quel point de l'ensemble des candidats par l'équation

$$\sigma_i^2 = \frac{1}{|L|} \sum_{\ell=1}^{L} \left( y_i^{\ell} - \bar{y}_i \right)^2 \ (4)$$

où $\quad \bar{y}_i = \dfrac{1}{|L|} \sum_{\ell=1}^{L} y_i^{\ell}$ est la moyenne des prédictions des L modèles au point $\boldsymbol{x}_i$.

**Variantes**

Selon un mode de réalisation, la sélection du sous-ensemble est réalisée selon une planification expérimentale. Selon ce mode de réalisation, on sélectionne d'autres composés moléculaires si le critère est conforme à un seuil donné. En d'autres termes, on enrichit le sous-ensemble de nouveaux hydrocarbures, pour améliorer la prédictivité du modèle.

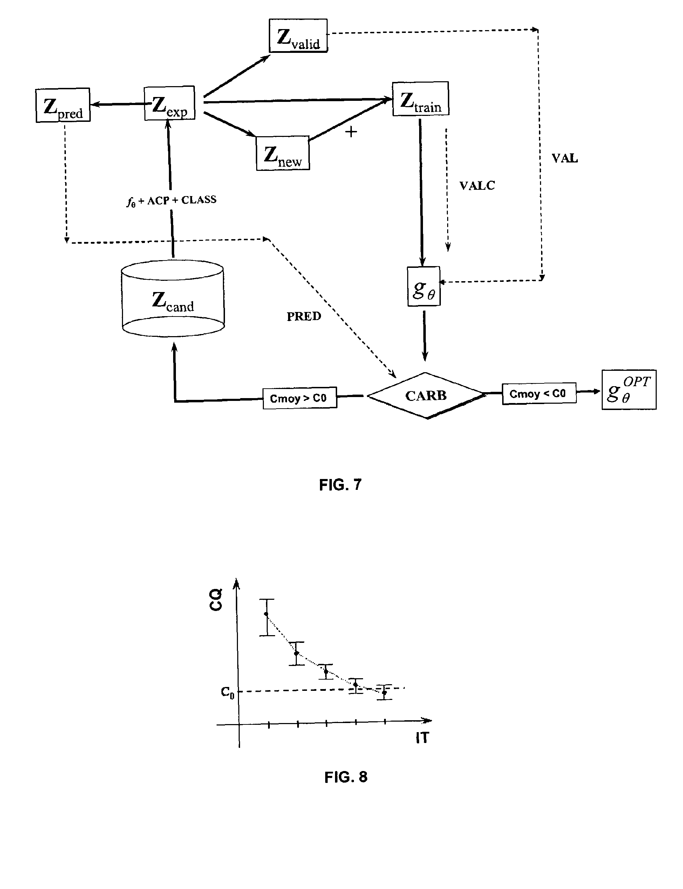

La figure 7 représente la boucle de planification expérimentale pour la construction d'un modèle GM ("*Graphe Machine*") optimal $\left( g_{\theta}^{OPT} \right)$. Les ensembles $\boldsymbol{Z}_{cand}$, $\boldsymbol{Z}_{exp}$, $\boldsymbol{Z}_{train}$, $\boldsymbol{Z}_{valid}$ et $\boldsymbol{Z}_{pred}$ sont définis ci-dessous.

Base de données (BdD) : $\boldsymbol{Z}_{cand} = \{\boldsymbol{x}_i, i = 1, ..., n_{cand}\}$ : ensemble de $n_{cand}$ individus candidats à l'expérimentation (les valeurs de la réponse $\boldsymbol{y}$ ne sont pas disponibles).

Base d'expérimentation : $\boldsymbol{Z}_{exp} = \{(\boldsymbol{x}_i, y_i), i = 1, ..., n_{exp}\}$ ensemble d'expériences réalisées pour construire le modèle. Cet ensemble peut être partagé en 3 ensembles Base d'apprentissage : $\boldsymbol{Z}_{train} = \{(\boldsymbol{x}_i, y_i), i = 1, ..., n_{train}\}$ ensemble de $n_{train}$ expériences qui servent à estimer les paramètres $\theta$ du modèle

Base de validation : $\boldsymbol{Z}_{valid}$ ensemble de $n_{valid}$ expériences servant à "ajuster" ces paramètres pour éviter d'obtenir un modèle "sur appris". Cette base est optionnelle dans la modélisation puisqu'il est possible de valider un modèle par la méthode du leave-one-out réel ou virtuel.

Base de prédiction : $\boldsymbol{Z}_{pred}$ ensemble de $n_{pred}$ expériences servant à tester le modèle final obtenu.

On a : $n_{exp} = n_{train} + n_{valid} + n_{pred}$

On définit un critère d'arrêt (CARB) de la boucle ci-dessus basé sur la qualité du modèle obtenu à une itération donnée. La qualité d'un modèle peut être définie de deux manières : en se basant sur la capacité prédictive du modèle, ou sur sa variance de prédiction, ou sur les deux à la fois (selon les besoins de l'utilisateur). On peut aussi tenir compte de contraintes économique telles que le nombre totale d'expériences à réaliser ou le nombre d'itérations à effectuer avant d'avoir un modèle optimal.

**Capacité prédictive d'un modèle** : deux critères peuvent rendre compte de la capacité de prédiction d'un modèle : l'erreur quadratique de prédiction (RMSEP) et le $R^2$ calculés à partir de l'ensemble de données de prédiction. Ces deux critères ont été définis en tant que distributions par les formules (1) et (2) ci-dessus à partir de L modèles. On pourra calculer pour chacun des deux critères une valeur moyenne qu'on définira respectivement par

$$\text{RMSE}_{moy} = \frac{1}{|L|} \sum_{\ell=1}^{L} \text{RMSE}_{pred}^{\ell} \quad (5)$$

et

$$R_{moy}^{2} = \frac{1}{|L|} \sum_{\ell=1}^{L} Q_{pred}^{\ell}{}^{2} \tag{6}$$

**Écart-type de prédiction d'un modèle** : un autre critère pouvant caractériser la qualité d'un modèle est celui rattaché à son écart-type de prédiction $\sigma_i$ (i.e. sa précision) défini par la formule (4) ci-dessus pour toute charge de l'ensemble des expériences candidates. On peut calculer un écart-type moyen de prédiction à chaque itération de la boucle de planification adaptative par

$$\sigma_{moy} = \frac{1}{|n_{cand}|} \sum_{i=1}^{n_{cand}} \sigma_i \tag{7}$$

Les distributions des critères définis par les formules (1), (2) et (4) peuvent être représentées à l'aide de courbes. La figure 8 représente des courbes des distributions du critère de qualité (CQ) du modèle construit à chaque itération (IT) de la boucle de planification adaptative. La barre horizontale indique une valeur $C_0$ d'un critère seuil défini en tant que critère d'arrêt de la boucle par l'utilisateur. Ainsi l'itération d'arrêt de la boucle est celle pour laquelle $C_{moy} \leq C_0$ (ou $C_{moy} \geq C_0$ selon la nature du critère). Ici le critère est à minimiser (ex. RMSEP ou écart-type de prédiction).
Un exemple d'algorithme de planification adaptative est présenté ci-après (figure 7).

Définition des critères mathématiques de fonctionnement de l'algorithme

- Fixer une valeur seuil $C_0$ pour un ou plusieurs critères parmi ceux définis par les égalités (5), (6) et (7).

- Postuler un modèle GM $f_\theta$ dont le nombre de paramètres est égal à $p$

Définition des exigences économiques liées à l'expérimentation

- Fixer $n_{train}$, $n_{valid}$ et $n_{pred}$ : nombres respectifs de charges dans les ensembles $\mathbf{Z}_{train}$ d'apprentissage, (éventuellement) $\mathbf{Z}_{valid}$ de validation et $\mathbf{Z}_{pred}$ de prédiction. (Pour former ces 3 ensembles, on peut appliquer la règle 60%+20%+20% ou 80%+10%+10% selon le cas)
  L'ensemble des expériences à réaliser est $\mathbf{Z}_{exp} = \mathbf{Z}_{train} \cup \mathbf{Z}_{valid} \cup \mathbf{Z}_{pred}$ dont l'effectif est égal à $n_{exp} = n_{train} + n_{valid} + n_{pred}$.
  Remarque : le nombre $n_{train}$ d'expériences de l'ensemble d'apprentissage doit être strictement supérieur à p.

- Fixer le nombre K des nouvelles charges à expérimenter à chaque itération de l'algorithme.

- Fixer (éventuellement) un nombre $n_{min}$ d'expériences à ne pas dépasser au bout de toutes les itérations de l'algorithme (coût économique).

- Fixer (éventuellement) un nombre $R_{min}$ d'itérations à ne pas dépasser (coût lié au temps passé sur les expérimentations).

Itération 0 de sélection des charges pour la construction d'un modèle initial

Sélection de 3 ensembles : $\mathbf{Z}_{train}$ d'apprentissage, $\mathbf{Z}_{valid}$ de validation et $\mathbf{Z}_{pred}$ de prédiction contenant respectivement $n_{train}$, $n_{valid}$ et $n_{pred}$ charges (l'ensemble $\mathbf{Z}_{valid}$ est optionnel). La sélection de ces 3 ensembles se fait de la manière suivante :

- Effectuer une ACP

- Effectuer 3 classifications (CLASS) différentes de l'ensemble $\mathbf{Z}_{cand}$ des candidats en

  ○ $n_{train}$ classes pour former l'ensemble $\mathbf{Z}_{train}$ d'apprentissage.

  ○ $n_{valid}$ classes pour former l'ensemble $\mathbf{Z}_{valid}$ de validation

  ○ $n_{pred}$ classes pour former l'ensemble $\mathbf{Z}_{pred}$ de prédiction
  On a $n_{exp} = n_{train} + n_{valid} + n_{pred}$

     - Représentations graphiques des plans factoriels et arbre de classification

- Sélectionner $n_{train}$ charges à expérimenter dans $\mathbf{Z}_{cand}$ afin de former $\mathbf{Z}_{train}$ : on sélectionne une charge par classe (charge la plus proche du centre de gravité de la classe)

- Sélectionner $n_{valid}$ charges à expérimenter dans $\mathbf{Z}_{cand}$ afin de former $\mathbf{Z}_{valid}$ : on sélectionne une charge par classe (soit de façon aléatoire soit en se basant sur des considérations métier)

- Sélectionner $n_{pred}$ charges à expérimenter dans $\mathbf{Z}_{cand}$ afin de former $\mathbf{Z}_{pred}$: on sélectionne une charge par classe (soit de façon aléatoire soit en se basant sur des considérations métier)

- Soit $\mathbf{Z}_{exp}$ l'ensemble des charges ainsi sélectionnées, on a $\mathbf{Z}_{exp}=\mathbf{Z}_{train} \cup \mathbf{Z}_{valid} \cup \mathbf{Z}_{pred}$.

- Réaliser les expérimentations des $n_{exp}$ charges de $\mathbf{Z}_{exp}$ : obtention des $n_{exp}$ valeurs $\mathbf{y}_i$ de la réponse $\mathbf{y}$ à modéliser.

<u>Incrémentation des itérations de l'algorithme de planification adaptative à partir des ensembles $\mathbf{Z}_{train}$, $\mathbf{Z}_{valid}$ et $\mathbf{Z}_{pred}$,</u>

Tant que $C_{moy} > C_0$, effectuer les opérations suivantes :

Etape 1. Construire plusieurs modèles sur l'ensemble d'apprentissage $\mathbf{Z}_{train}$ et en choisir L optimaux $f_{\hat{\theta}}^{\ell}$ par validation (leave-one-out réel, virtuel ou à partir de $\mathbf{Z}_{valid}$)

Etape 2. Appliquer les L modèles sur les 2 ensembles

- $\mathbf{Z}_{pred}$ de prédiction : calcul de $RMSEP_{pred}^{\ell}$ et $R_{pred}^{\ell\,2}$

- $\mathbf{Z}_{cand}$ des charges candidates : calcul de $\sigma_i$ pour toute charge candidate i

Etape 3. Représenter graphiquement les courbes des critères en fonction des itérations

Etape 4. Effectuer une ACP suivie d'une classification de l'ensemble $\mathbf{Z}_{cand}$ en K classes $C_k$ (pour k = 1, K)

Etape 5. Représentation graphique des plans de l'ACP et de l'arbre de classification

Etape 6. Constituer un nouvel ensemble $\mathbf{Z}_{new}$ formé de K charges sélectionnées dans $\mathbf{Z}_{cand}$ en y incluant une charge i par classe $C_k$ telle que $\sigma_i = \max_{i' \in C_k} \sigma_{i'}$

Etape 7. Former un nouvel ensemble d'apprentissage $\mathbf{Z}_{train}$ par union du précédent avec $\mathbf{Z}_{new}$ : $\mathbf{Z}_{train} \leftarrow \mathbf{Z}_{train} \cup \mathbf{Z}_{new}$

Etape 8. Réaliser les expérimentations des $n_{new}$ charges de $\mathbf{Z}_{new}$ : obtention des $n_{new}$ valeurs $\mathbf{y}_i$ de la réponse $\mathbf{y}$ à modéliser.

Revenir à Étape 1.
Le nombre total $n_{total}$ d'expériences réalisées, au bout de R itérations, à raison de K nouvelles expériences par itération, est égal à

$$n_{total} = n_{train} + n_{valid} + n_{pred} + R.K$$

L'utilisateur peut éventuellement décider d'interrompre l'algorithme afin que ce nombre ne dépasse pas un nombre minimal $n_{min}$ d'expériences ou un nombre minimal $R_{min}$ à ne pas dépasser préalablement fixés pour des raisons économiques.

5. Utilisation du modèle de réponses

Le modèle de réponses construit aux étapes 1 à 4 permet de réaliser les analyses suivantes, cette liste étant non exhaustive :

a. prédiction du comportement d'un processus expérimental ;

b. amélioration du fonctionnement d'un processus expérimental ;

c. explication des mécanismes de fonctionnement d'un processus expérimental ;

d. identification de molécules ayant une propriété donnée dans la base de données.

e. estimation de propriété physique ou chimique d'un composé moléculaire connaissant sa structure moléculaire. Pour ce faire, il suffit de construire une fonction paramétrée pour ce composé moléculaire, par la méthode des "Graphe Machines", puis d'utiliser cette fonction avec les paramètres θ déterminés grâce à la base de données (et pour la même propriété). La réponse de la fonction paramétrée, donnant la valeur de la propriété.

**Exemple d'utilisation du modèle pour la modélisation de l'indice de cétane d'hydrocarbures**

La base de données disponible est constituée de 146 hydrocarbures. On souhaite modéliser la réponse *Indice de Cétane* à partir de cette base. Nous souhaitons optimiser le choix de la base d'apprentissage, c'est-à-dire de l'ensemble des données utilisées pour estimer un modèle final ayant les meilleures capacités de généralisation (i.e. un modèle donnant des bonnes prédictions pour des données non utilisées pour le construire).

**Analyse factorielle :**

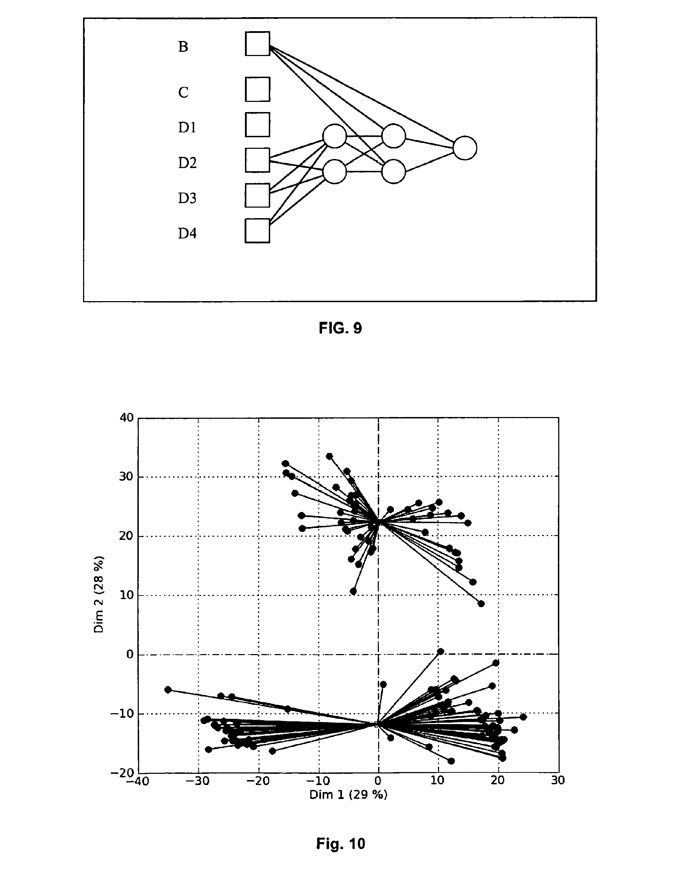

On construit les fonctions paramétrées ("*Graph Machines*") des 146 hydrocarbures. On choisit pour cela comme fonction élémentaire de noeud des réseaux de neurones à 2 couches de neurones cachés. De plus, les hydrocarbures n'étant composés que de carbone et d'hydrogène, un seul atome non-hydrogène est possible. Les réseaux de neurones utilisés ne comportent donc que 5 entrées (c'est-à-dire une pour l'atome de carbone (C), et 4 pour les 4 degrés (D1 à D4) possibles de cet atome). La figure 9 représente ainsi la structure de la fonction élémentaire choisie pour l'analyse factorielle. L'entrée B, représente le biais introduit pour la modélisation. Ce réseau élémentaire contient donc 15 paramètres ajustables.

Afin d'effectuer la sélection des molécules les plus pertinentes pour ajuster le modèle, c'est-à-dire le mieux réparties possible, il est nécessaire de définir une distance entre les molécules, donc entre les graph machines. Nous choisissons pour cela un ensemble de $10^3$ vecteurs de 19 paramètres aléatoirement, entre les valeurs -1 et 1. Les valeurs prises par chacune de 146 graph machines pour ces différents paramètres sont alors calculées. Chaque molécule est alors représentée par un vecteur de $10^3$ paramètres, et la comparaison de ces vecteurs permet de mesurer une distance entre ces molécules. La dimension des vecteurs est réduite par une analyse en composantes principales (ACP), ce qui permet à la fois de visualiser les molécules dans un espace à deux dimensions (qui sont les deux premières composantes principales par exemple) et de classifier ces molécules selon leur proximité.

La figure 10 représente ainsi les images des 146 hydrocarbures dans le plan des deux composantes principales (Dim1 et Dim2) obtenues par ACP, représentant respectivement 29% et 28% de l'information. Les 10 premières composantes principales en représentent 92%. Une classification permet alors de distinguer deux classes principales de molécules. Les molécules sont représentées sur la figure 10 par des points reliés au centre d'inertie de leur classe.

**Planification expérimentale :**

Le but de cette planification expérimentale est de sélectionner un minimum de molécules à expérimenter, permettant d'obtenir un modèle optimal. Ce modèle est ajusté à l'aide d'exemples constituant la base d'apprentissage, pour lesquels l'indice de cétane doit être connu. La méthode développée consiste à sélectionner dans un premier temps une base d'apprentissage de petite taille (nous avons choisi 30% c'est-à-dire 44 molécules), puis de compléter cette base par étapes.

### Étape 1 :

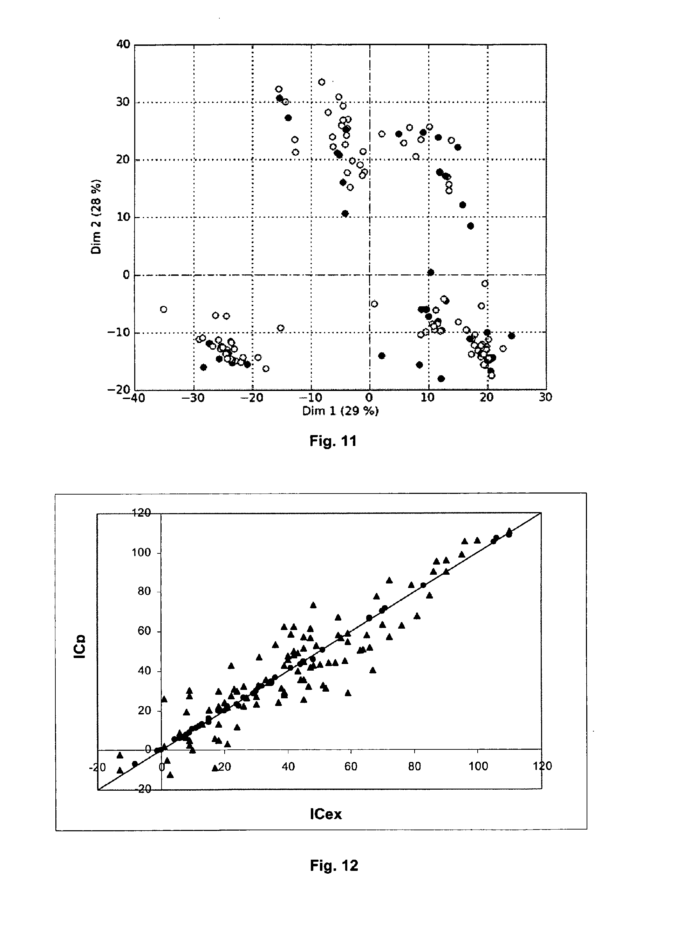

Les molécules de la base d'apprentissage doivent être représentatives de la base entière : elles doivent donc être bien réparties dans l'espace. On utilise les résultats de l'analyse factorielle pour sélectionner cette base. La figure 11 représente ainsi les exemples de la base d'apprentissage (points noirs et de la base de prédiction (points blancs), dans le plan des deux premières composantes principales de l'ACP (Dim1 et Dim2). On constate effectivement que les molécules sont uniformément réparties dans ce plan.

Le modèle est alors établi de la façon suivante :

- les graph machines associées aux 44 molécules de la base d'apprentissage sont construites, avec pour fonction de noeud élémentaire des réseaux de neurones à 4 neurones cachés

- une série de 20 apprentissages est réalisée. Les paramètres des graph machines sont calculés avec 25 initialisations différentes, de façon à minimiser l'erreur du modèle par rapport aux valeurs expérimentales de l'indice de cétane.

- un modèle est alors sélectionné : c'est la moyenne des 6 modèles permettant d'obtenir le plus faible score de leave-one-out virtuel. Cette sélection permet d'assurer que le modèle choisi a de bonnes capacités de généralisation.

- les valeurs de l'indice de cétane obtenues par ce modèle sur la base d'apprentissage sont calculées.

- les valeurs de l'indice de cétane des exemples de la base de prédiction sont également calculées à partir de ce modèle, ainsi que les écarts-types des prédictions fournies par les 6 modèles sélectionnés. A chaque exemple de la base de prédiction est donc associée une valeur prédite, et un écart-type. On calcule également la moyenne de ces écarts-types. Plus cette moyenne est faible, et plus les prédictions sont fiables. Les valeurs apprises et prédites sur les bases d'apprentissage et de prédiction sont représentées sur la figure 12, où l'axe des abscisses représente l'indice de cétane expérimental (ICex), et l'axe des ordonnées représente l'indice de cétane prédit (ICp). Les triangles correspondent aux valeurs issues de la base de prédiction, et les ronds correspondent aux valeurs issues de la base d'apprentissage.

- Pour améliorer le modèle, la base d'apprentissage peut être étendue. On ajoute alors à cette base 7 exemples supplémentaires (soit environ 5% de la base totale), choisis selon deux critères : ils doivent être représentatif de la base des hydrocarbures (c'est-à-dire être bien répartis dans l'espace des composantes principales), et avoir un écart-type de prédiction important (il s'agit des exemples dont la prédiction de l'indice de cétane est la moins fiable). Une analyse factorielle est réalisée pour sélectionner ces 7 exemples en tenant compte de ces critères. La base d'apprentissage comporte alors 44+7 exemples.

### Étape *n* :

A l'étape *n*, la base d'apprentissage est constituée de 44+7*n* exemples. Les paramètres du modèle sont ajustés selon la même méthode que lors de l'étape 1. Les prédictions du modèle et les écarts-types de ces prédictions sont calculées. Si la moyenne de ces écarts-types est inférieure à la moyenne des écarts-types de l'étape n-1, le modèle a été amélioré. La planification expérimentale continue donc, avec l'ajout de 7 molécules, ce qui conduit à l'étape n+1.

### Arrêt de la boucle :

Lorsque l'écart-type des prédictions ne diminue plus, on peut estimer que l'ajout de nouveaux exemples à la base d'apprentissage n'apporte plus d'information au modèle, et ne permet pas de le rendre plus précis. La boucle de planification expérimentale est alors stoppée.

Le tableau 1 résume la variation des erreurs de prédiction (erreur quadratique moyenne), ainsi que l'écart-type moyen des prédictions, lors des étapes 1 à 11.

Tableau 1 : Variation de l'écart-type des prédictions et de l'erreur moyenne lors des 11 itérations de la boucle de planification expérimentale

| Itération | Nombre exemples app. | Erreur moyenne d'apprentissage | Écart-type | Erreur moyenne de prédiction |
|---|---|---|---|---|
| 1 | 44 | 0.87 | 20.31 | 11.67 |
| 2 | 51 | 1.3 | 16.55 | 11.91 |
| 3 | 58 | 1.7 | 11.73 | 11.81 |
| 4 | 65 | 2.6 | 10.36 | 11.50 |
| 5 | 72 | 2.9 | 9.59 | 11.35 |
| 6 | 79 | 2.8 | 8.07 | 10.27 |
| 7 | 86 | 3.9 | 7.13 | 9.40 |
| 8 | 93 | 4.5 | 6.89 | 8.37 |
| 9 | 100 | 4.1 | 5.96 | 7.79 |
| **10** | **107** | **4.2** | **5.49** | **5.87** |
| 11 | 114 | 4.3 | 5.59 | 7.06 |

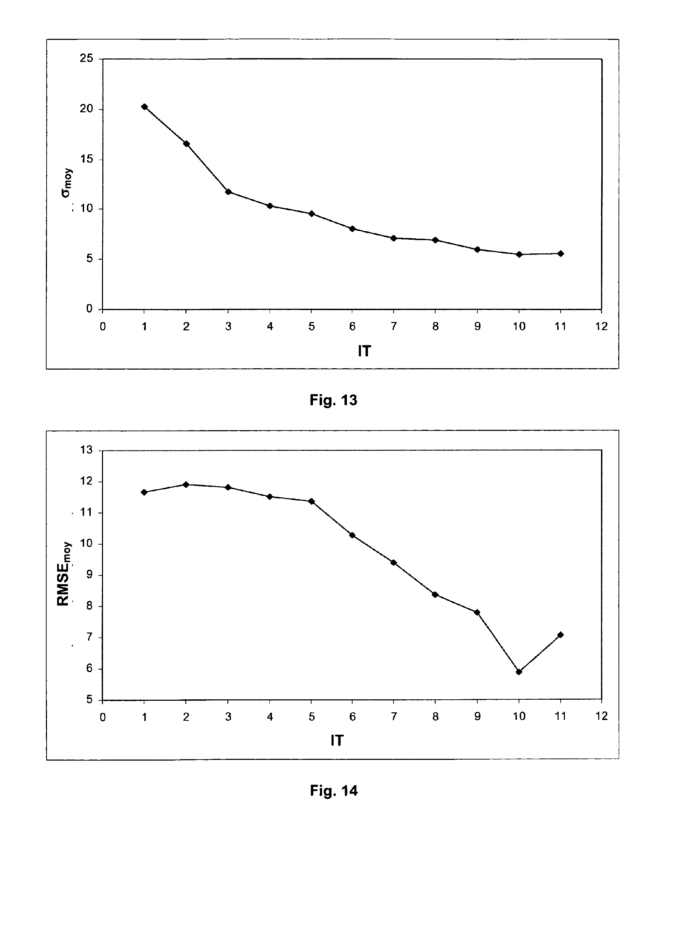

Ces résultats sont également représentés sur les figures 13 et 14. La figure 13 illustre la variation de l'écart-type des prédictions ($\sigma_{moy}$) en fonction des itérations (IT). La figure 14 illustre la variation de l'erreur quadratique moyenne de prédiction ($RMSE_{moy}$) en fonction des itérations (IT).

L'écart-type des prédictions ne diminue donc plus après la 10$^{ème}$ itération, de même que l'erreur absolue moyenne de prédiction : cela signifie que l'ajout de nouveaux exemples dans la base d'apprentissage n'améliore pas le modèle.

Le modèle optimum est ainsi obtenu à l'itération 10, ce qui correspond à une base d'apprentissage de 107 exemples et une base de prédiction de 39 molécules (soit respectivement 70% et 30% de la base de données pour l'apprentissage et la prédiction).

Ce modèle permet d'obtenir de très bons résultats à la fois en apprentissage et en prédiction, puisque les erreurs quadratiques moyennes sur ces deux bases sont respectivement égales à 4,2 et 5,87 points de cétane, c'est-à-dire très proches de l'erreur expérimentale estimée à 4 points de cétane.

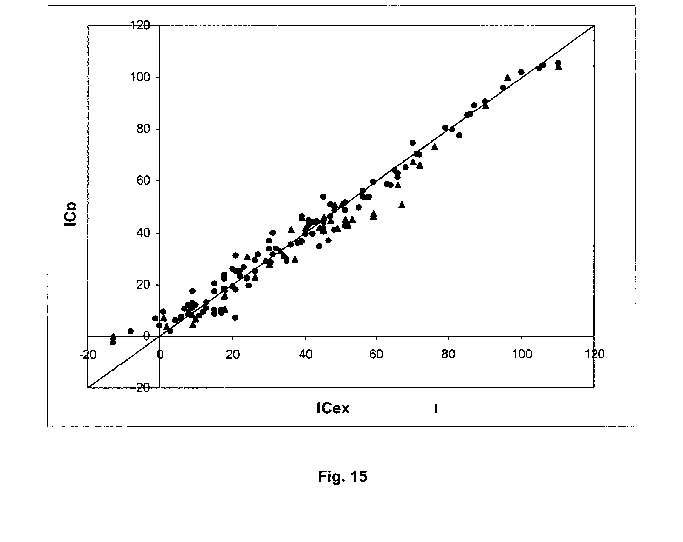

La figure 15 représente les valeurs estimées par le modèle sur l'ensemble de ces deux bases à la dixième itération : l'axe des abscisses représente l'indice de cétane expérimental (ICex), et l'axe des ordonnées représente l'indice de cétane prédit (ICp). Les triangles correspondent aux valeurs issues de la base de prédiction, et les ronds correspondent aux valeurs issues de la base d'apprentissage.

**Annexe**

**Codage de molécules par *"Graph Machines"***

**Codage d'une molécule par un graphe**

Une molécule chimique est représentée par un graphe de la manière suivante :

- les atomes d'hydrogène ne sont pas directement pris en compte ;

- chaque atome non-hydrogène est représenté par un noeud ;

- chaque liaison entre deux atomes, quelle que soit sa nature (liaison simple, double, triple ou aromatique) est représentée par une arête simple entre les noeuds correspondants à ces deux atomes.

A chacun des noeuds du graphe ainsi obtenu est associée une étiquette, caractérisant l'atome correspondant. Les informations portées par ces étiquettes peuvent être de plusieurs natures. Il peut s'agir :

- d'informations sur l'atome, telles que :

  ○ sa nature chimique (carbone, azote, oxygène...),

  ○ son degré, c'est-à-dire le nombre de liaisons formées par cet atome avec d'autres atomes, autres que l'hydrogène,

  ○ sa stéréochimie, par exemple sa configuration Z/E ou R/S,

  ○ toute autre information supplémentaire telle que sa charge ponctuelle...

- de caractéristiques globales de la molécule, telles que des descripteurs associés à cette molécule,

- de valeurs numériques associées aux grandeurs fixées par l'utilisateur ou mesurées dans le système (paramètres du processus, propriétés des charges en entrée, concentrations des constituants, conditions expérimentales, paramètres fonctionnels...),

- de catégories associées à la molécule telle que sa famille chimique ou son appartenance à une classe quelconque

- ...

La figure 2 présente un exemple de codage de molécule par un graphe. Les étiquettes portées par chacun des noeuds indiquent la nature de chaque atome ainsi que son degré. Par exemple, l'atome d'azote (N) ne forme qu'une liaison avec un atome autre que l'hydrogène, il est donc de degré 1. L'étiquette portée par le noeud correspondant indique ainsi qu'il s'agit d'un atome d'azote de degré 1 (étiquette N, 1).

**Transformation d'un graphe quelconque en graphe acyclique orienté**

Les graphes obtenus à partir des molécules sont des graphes non orientés, qui peuvent comporter un ou plusieurs cycles. Ces graphes doivent être transformés en *arborescences,* c'est-à-dire en graphes acycliques orientés, dont toutes les arêtes convergent vers la *racine.* Cette conversion est nécessaire pour la construction ultérieure des Graph Machines à partir des graphes. Elle se déroule selon les étapes suivantes :

- Le noeud racine du graphe est tout d'abord choisi. Il s'agit du noeud central de la molécule, c'est-à-dire celui dont la distance maximale aux autres noeuds est la plus faible. Si deux noeuds présentent la même distance maximale, celui de plus haut degré est choisi. Si ces degrés sont également les mêmes, les atomes adjacents à ces deux noeuds sont à leur tour comparés.

- Si le graphe est cyclique, chaque cycle est ouvert. Il faut pour cela supprimer autant d'arêtes qu'il y a de cycles. Pour un cycle donné, l'arête supprimée est celle la plus éloignée du noeud racine

- Les arêtes sont finalement orientées, de manière à toutes diverger depuis le noeud racine.

- La figure 3 représente la conversion du graphe exemple de la figure 2 en arborescence.

Dans l'exemple de la figure 3, les noeuds *1* et *2* sont tous deux à une distance maximale de 3 arêtes des autres noeuds. Cependant, le noeud *1* est de degré 4 tandis que le noeud *2* est de degré 3 : c'est donc le noeud 1 qui est choisi comme noeud racine de l'arborescence. L'arête reliant les noeuds *3* et *4* est supprimée pour rendre le graphe acyclique, et les arêtes sont orientées à partir du noeud racine vers les noeuds feuilles de l'arborescence.

**Construction des *Graph Machines* à partir des arborescences**

A chaque arborescence est associée une fonction mathématique, appelée *Graph Machine,* dont la structure reproduit celle de l'arborescence. On définit une fonction élémentaire $f_\theta$, paramétrée par le vecteur de paramètres θ. Cette fonction peut être une fonction linéaire, un polynôme, ou un réseau de neurones par exemple.
A chaque noeud de l'arborescence est associée cette fonction $f_\theta$, appelée pour cette raison *fonction de noeud,* où θ est identique pour tous les noeuds.

Pour chaque graphe $G_i$, on construit alors une fonction $g_\theta^i$ par composition des fonctions $f_\theta$, de façon à refléter la structure du graphe : si $s_a$ et $s_b$ sont deux sommets du graphe, tels que *a* est parent de *b* (i.e. un arc part de $s_a$ et arrive en $s_b$), alors le résultat de la fonction associée au noeud $s_b$ est argument de celle associée au noeud $s_a$.

La fonction de noeud paramétrée $f_\theta$ associée au noeud z est donc de la forme :

$$f_\theta(z) = f(u,v)$$

où :

- u est un vecteur dont les composantes sont égales aux arguments de sorties des fonctions associées aux noeuds parents du noeud z en question

- v est un vecteur optionnel dont les composantes fournissent l'information localisée au noeud : ce sont les étiquettes du noeud pouvant être une valeur qualitative (comme la nature du noeud) ou quantitative (comme les descripteurs associés à la molécule).

Finalement, la sortie de la fonction $g_\theta^i$ est la sortie de la fonction associée au noeud racine.

La figure 4 représente ainsi une molécule, l'arborescence associée, et la *Graph Machine* construite à partir de cette arborescence. La sortie de cette *Graph Machine* est celle de la fonction associée au noeud 1. Cette fonction prend pour argument les sorties des fonctions associées aux noeuds 2, 3 et 4, qui sont les enfants du noeud 1, ainsi que les informations portées par l'étiquette du noeud 1, c'est-à-dire la nature de l'atome correspondant et son degré.

**Codage d'un mélange de molécules par un graphe :**

Le codage d'une molécule par un graphe peut être étendu à un mélange de deux ou plusieurs molécules. A chacune des molécules du mélange est associé un graphe, dont chacun des noeuds porte une étiquette indiquant la proportion de la molécule dans le mélange. Les deux graphes sont alors reliés par leur noeud racine, par l'intermédiaire d'un noeud supplémentaire, qui devient le noeud racine du graphe représentant le mélange. La construction de la *Graph Machine* associée au mélange est effectuée selon la même méthode que pour une molécule simple.

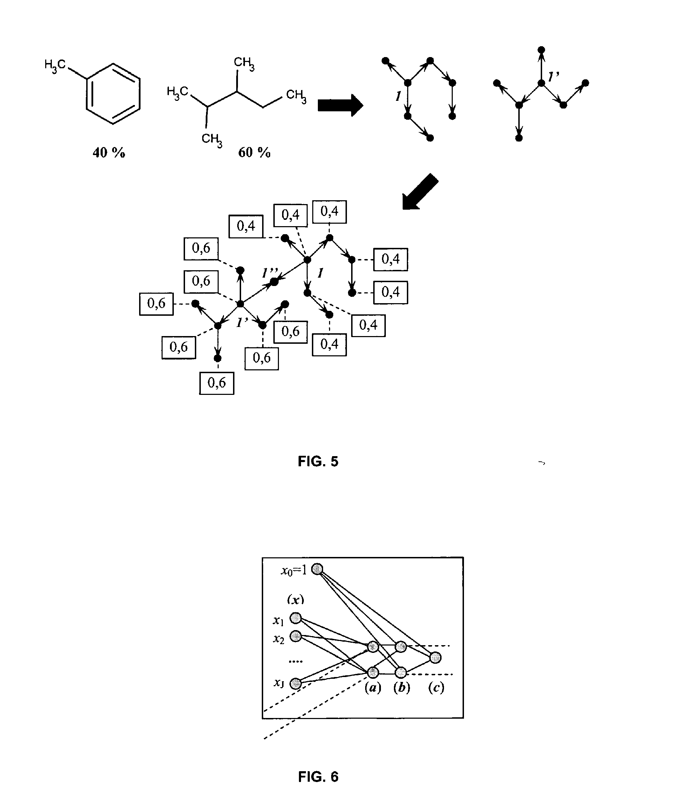

La figure 5 représente un exemple de codage d'un mélange de deux molécules par un graphe unique.

Les deux molécules représentées sont présentes dans le mélange selon les proportions 40% et 60%. Dans une première étape, les molécules sont converties en arborescences, de noeuds racines *1* et *1'*. Ces arborescences sont reliées par l'intermédiaire d'un noeud supplémentaire, *1''*, qui est alors parent des noeuds *1* et *1'*. Ce noeud *1''* est ainsi le noeud racine de l'arborescence obtenue.

**Structure de la fonction élémentaire : réseau de neurones**

Notations

On désigne par $\boldsymbol{x} = (x_0, x_i, x_2, ..., x_J, x_{J+1},...x_{J+J''})$ le vecteur des entrées de la fonction élémentaire *f*.

$x_0$ est la variable qui prend la valeur 1 associée au biais du modèle (ou à la constante dans le cas où *f* est un modèle polynomial). J est le nombre d'étiquettes définies aux noeuds du graphe et J' le nombre maximum d'enfants pouvant être admis pour un noeud sur l'ensemble de toutes les molécules de la base de données.

Les variables $x_j$ (pour j=1,J) peuvent être

- la nature de l'atome du noeud (variable indicatrice),

- le nombre de liaisons (variable indicatrice),

- des variables externes considérées comme des étiquettes associées à tous les noeuds du graphe. Ces variables peuvent être

  ■ des variables indicatrices comme, par exemple, la famille chimique de la molécule ou tout autre variable de classification,

■ des variables quantitatives : conditions expérimentales (température, pression, concentration, ...), descripteurs moléculaires, paramètres fonctionnels, composantes principales obtenues par ACP, ...

Les variables notées $x_{J+1}, x_{J+2}, ..., x_{J+J'}$ correspondent aux sorties des J' noeuds enfants du noeud considéré. J' est le nombre maximum de noeuds enfants pouvant être admis (observé pour toutes les molécules de la base de données). Si un noeud possède moins de J' noeuds enfants, les sorties pour lesquelles il n'existe pas de noeud enfant sont nulles.

La structure de la fonction élémentaire lorsqu'il s'agit d'un réseau de neurones est représentée sur la figure 6. Ce réseau possède J+1 entrées et K neurones cachés).

Soit $u_{jk}$ (pour j=1,J et k=1,K) les poids connectant les entrées $x_j$ à la 1$^{ère}$ couche cachée du réseau notés $a_k$, $w_{0\ell}$ (pour $\ell$=1,K) les poids connectant $x_0$ à la 2$^{ème}$ couche cachée notés $b_\ell$, $w_{k\ell}$ (pour k=1,K et $\ell$=1,K) les poids connectant les neurones de la 1$^{ère}$ couche cachée à ceux de la 2$^{ème}$ couche cachée, et $v_l$ (pour $\ell$=1,K) les poids connectant les neurones de la 2$^{ème}$ couche cachée au neurone de sortie c. On a :

$$a_k = \sum_{j=1}^{J} u_{jk} x_j + \sum_{j=J+1}^{J+J'} f_\theta^{jk} \quad \text{pour k=1,K}$$

où $f_\theta^{jk}$ est la sortie de l'enfant j du noeud k (celui-ci peut au maximum admettre J' enfants). et

$$b_\ell = h\left( w_{0\ell} + \sum_{k=1}^{K} w_{k\ell} a_\ell \right) \text{ pour } \ell\text{=1,K}$$

où h est la fonction tangente hyperbolique définie par

$$h(z) = \frac{e^z - e^{-z}}{e^z + e^{-z}}$$

Lorsque la fonction est associée à un noeud feuille ou branche, les sorties des neurones de la seconde couche cachée sont à leur tour les entrées de la fonction du noeud parent. Ces sorties sont représentées sur la figure 6 par des traits discontinus. Le neurone *c* est alors absent.

Par contre, lorsqu'il s'agit du noeud racine, le réseau comporte un neurone supplémentaire, dit de sortie : la sortie de ce neurone *c* est alors la sortie de la *Graph Machine* correspondante. Elle vaut :

$$c = g_\theta = v_0 + \sum_{\ell=1}^{K} v_\ell b_\ell$$

Le vecteur θ des paramètres est constitué par les poids *u, w* et *v* des connections. Le nombre de ses composantes est : $p = J.K + K + K^2 + K + 1$

**Revendications**

**1.** Procédé pour déterminer une propriété physique ou chimique d'un composé moléculaire de structure moléculaire connue, à partir d'une base de données constituée d'autres composés moléculaires de structures moléculaires connues, **caractérisé en ce que** :

a- on sélectionne dans ladite base de données, un sous-ensemble de composés moléculaires représentatif d'une variabilité des structures moléculaires des composés de ladite base de données, au moyen d'une analyse descriptive multidimensionnelle desdites structures moléculaires ;

b- et pour chaque composé moléculaire *C* dudit sous-ensemble

-- on construit, au moyen d'une technique de "Graph Machines", une fonction paramétrée $g_\theta$ définie en fonction de paramètres constituant un vecteur θ, et chaque fonction paramétrée $g_\theta$ ayant une forme mathématique traduisant la structure moléculaire dudit composé moléculaire *C* ;

- on mesure expérimentalement ladite propriété dudit composé moléculaire *C* ;

c- on estime une valeur pour chaque paramètre dudit vecteur θ, de façon à ce que pour chaque composé moléculaire *C*, la fonction paramétrée $g_\theta$ donne un résultat sensiblement égale à ladite mesure expérimentale correspondante ;

d- on détermine ladite propriété physique ou chimique dudit composé moléculaire, en construisant une fonction paramétrée pour ce composé moléculaire et en utilisant lesdits paramètres θ déterminés à l'étape c.

2. Procédé selon la revendication 1, dans lequel on construit ladite fonction paramétrée $g_\theta$ d'un composé *c* au moyen des étapes suivantes :

- on construit un graphe acyclique orienté représentant une structure moléculaire dudit composé *c*, et comportant un ensemble de noeuds ;

- on définit une fonction élémentaire $f_\theta$ paramétrée par ledit vecteur θ de paramètres, et associée à chaque noeud dudit graphe ;

- on définit ladite fonction paramétrée $g_\theta$ par composition de ladite fonction $f_\theta$ en fonction dudit graphe de façon à refléter ladite structure moléculaire.

3. Procédé selon la revendication 2, dans lequel on construit un graphe acyclique orienté, de la façon suivante pour chaque molécule d'un composé moléculaire :

- chaque atome non hydrogène est représenté par un noeud, auquel est associé une étiquette caractérisant l'atome ;

- une liaison entre deux atomes est représentée par une arête simple entre les noeuds correspondants à ces deux atomes ;

4. Procédé selon l'une des revendications 2 et 3, dans lequel ladite fonction élémentaire $f_\theta$, est un polynôme ou un réseau de neurones.

5. Procédé selon l'une des revendications 2 à 4, dans lequel ladite fonction élémentaire $f_\theta$, est également fonction de propriétés liées à un atome associé au noeud auquel est associée ladite fonction élémentaire $f_\theta$, et fonction de propriétés liées à au moins une molécule dudit composé *c*.

6. Procédé selon la revendication 5, dans lequel les propriétés liées à l'atome sont choisies parmi les propriétés suivantes : nature chimique, degré, stéréochimie, charge ponctuelle ; et les propriétés liées à au moins une molécule sont choisies parmi les propriétés suivantes : descripteurs moléculaires, concentrations des composés, conditions expérimentales, paramètres fonctionnels, famille chimique, mesure de types quantitatif ou qualitatif associée à l'atome.

7. Procédé selon l'une des revendications précédentes, dans lequel on réalise ladite analyse descriptive multidimensionnelle en réalisant les étapes suivantes:

- on associe à chacun des *n* composés moléculaires de ladite base de données, une fonction paramétrée $h_\theta$ construite au moyen d'une technique de "Graph Machines" et définie en fonction de *p* paramètres du vecteur θ ;

- on effectue un tirage aléatoire d'un nombre M de vecteurs θ dans l'espace $\mathbf{R}^p$ des paramètres ;

- on projette chacun des *n* composés moléculaires dans l'espace $\mathbf{R}^M$, sous forme de nuage de *n* points dont les *M* coordonnées sont les résultats de ladite fonction paramétrée $h^i_\theta$ associée à chaque composé moléculaire noté i, pour i = 1 à n ; et
- on définit une distance entre deux points à partir de laquelle on évalue une proximité entre deux composés moléculaires.

**8.** Procédé selon la revendication 7, dans lequel on réduit la dimension de l'espace $\mathbf{R}^M$ en réalisant une analyse en composantes principales, et on sélectionne lesdits composés moléculaires au moyen d'une technique de classification des premières composantes significatives issues de ladite analyse en composantes principales.

**9.** Procédé selon l'une des revendications précédentes, dans lequel on définit ledit vecteur de paramètres θ, en définissant une fonction de coût mesurant des écarts entre lesdites mesures et lesdites valeurs prédites par ladite fonction paramétrée, et en minimisant ladite fonction de coût par modification itérative des paramètres.

**10.** Procédé selon la revendication 9, dans lequel :

- on répartit lesdits composés moléculaires dudit sous-ensemble en deux bases de données : une base d'apprentissage et une base de prédiction ;
- on détermine les paramètres du vecteur θ par minimisation de ladite fonction de coût en utilisant uniquement des composés moléculaires de ladite base d'apprentissage ;
- on définit un critère traduisant un caractère prédictif de ladite fonction paramétrée, à partir de ladite base de prédiction ;
- on modifie lesdits paramètres du vecteur θ de façon itérative jusqu'à ce que ledit critère soit conforme à un seuil donné.

**11.** Procédé selon la revendication 10, dans lequel on sélectionne d'autres composés moléculaires si ledit critère est conforme à un seuil donné.

**12.** Méthode selon l'une des revendications précédentes, dans laquelle chaque composé moléculaire comporte plusieurs molécules, et on construit un graphe acyclique orienté pour ledit composé moléculaire par assemblage de graphes acycliques orientés construits pour chacune desdites molécules.

**13.** Méthode selon l'une des revendications précédentes, dans laquelle lesdits composés moléculaires sont des hydrocarbures.

**14.** Méthode selon la revendication 13, dans laquelle ladite propriété physique ou chimique dudit composé moléculaire est l'indice de cétane desdits hydrocarbures.

PE

E → →  [Syst] → →  S

$x$ → →  $\boxed{f(x,\theta) + \varepsilon}$ → →  $y$

**FIG. 1**

**FIG. 2**

**FIG. 3**

$$g_\theta = f_\theta \left( f_\theta(z_2), f_\theta(z_3), f_\theta(z_4), (1,0,0,0), 3 \right)$$

**FIG. 4**

**FIG. 5**

**FIG. 6**

**FIG. 7**

**FIG. 8**

**FIG. 9**

**Fig. 10**

Fig. 11

Fig. 12

**Fig. 13**

**Fig. 14**

**Fig. 15**

Europäisches
Patentamt

European
Patent Office

Office européen
des brevets

**RAPPORT DE RECHERCHE EUROPEENNE**

Numéro de la demande

EP 11 29 0196

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X,D | GOULON A. ET AL: "Graph Machines and Their Applications to Computer-Aided Drug Design: A New Approach to Learning from Structured Data", UNCONVENTIONAL COMPUTATION LECTURE NOTES IN COMPUTER SCIENCE;;LNCS, SPRINGER, BERLIN, DE, vol. 4135, 1 janvier 2006 (2006-01-01), pages 1-19, XP019039711, ISBN: 978-3-540-38593-6 * le document en entier * ----- | 1-14 | INV. G06F19/00 |
| X,D | GOULON A. ET AL: "Predicting activities without computing descriptors: graph machines for QSAR", SAR AND QSAR IN ENVIRONMENTAL RESEARCH, vol. 18, no. 1-2, janvier 2007 (2007-01), pages 141-153, XP008132816, ISSN: 1062-936X * le document en entier * ----- | 1-14 | |
| X,D | GOULON-SIGWALT-ABRAM A. ET AL: "From Hopfield nets to recursive networks to graph machines: Numerical machine learning for structured data", THEORETICAL COMPUTER SCIENCE, AMSTERDAM, NL, vol. 344, no. 2-3, 17 novembre 2005 (2005-11-17), pages 298-334, XP005134953, ISSN: 0304-3975, DOI: DOI:10.1016/J.TCS.2005.08.026 * le document en entier * * en particulier:; page 325 - page 328 * ----- -/-- | 1-14 | **DOMAINES TECHNIQUES RECHERCHES (IPC)** G06F |

Le présent rapport a été établi pour toutes les revendications

| Lieu de la recherche | Date d'achèvement de la recherche | Examinateur |
|---|---|---|
| La Haye | 28 juillet 2011 | Godzina, Przemyslaw |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
...........................................................................................
& : membre de la même famille, document correspondant

EPO FORM 1503 03.82 (P04C02)

Europäisches
Patentamt

European
Patent Office

Office européen
des brevets

**RAPPORT DE RECHERCHE EUROPEENNE**

Numéro de la demande

EP 11 29 0196

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | LI H. ET AL: "Considerations and recent advances in QSAR models for cytochrome P450-mediated drug metabolism prediction", JOURNAL OF COMPUTER-AIDED MOLECULAR DESIGN, vol. 22, no. 11, novembre 2008 (2008-11), pages 843-855, XP002621440, ISSN: 0920-654X * abrégé * * page 849, colonne de droite, ligne 50 - page 850, colonne de gauche, ligne 35 * ----- | 1-14 | |
| A | IVANCIUC O.: "Weka machine learning for predicting the phospholipidosis inducing potential.", CURRENT TOPICS IN MEDICINAL CHEMISTRY 2008 LNKD- PUBMED:19075775, vol. 8, no. 18, 2008, pages 1691-1709, XP002621441, ISSN: 1873-4294 * page 1692, colonne de gauche, ligne 21-27 * ----- | 1-12 | |
| | | | DOMAINES TECHNIQUES RECHERCHES (IPC) |

Le présent rapport a été établi pour toutes les revendications

| Lieu de la recherche | Date d'achèvement de la recherche | Examinateur |
|---|---|---|
| La Haye | 28 juillet 2011 | Godzina, Przemyslaw |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **GOULON-SIGWALT-ABRAM ; A. DUPRAT ; G. DREYFUS.** From Hopfield nets to recursive networks to graph machines: Numerical machine learning for structured data. *Theoretical Computer Science,* 17 Novembre 2005, vol. 344 (2-3), 298-334
- **GOULON, T. PICOT ; A. DUPRAT ; G. DREYFUS.** Predicting activities without computing descriptors: graph machines for QSAR. *SAR and QSAR in Environmental Research,* Janvier 2007, vol. 18 (1, 2), 141-153
- **GOULON, A ; DUPRAT, G ; DREYFUS.** Graph Machines and Their Applications to Computer-Aided Drug Design: A New Approach to Learning from Structured Data. *Lecture Notes in Computer Science,* 2006, vol. 4135, 1-19
- **GOULON-SIGWALT-ABRAM ; A. DUPRAT ; G. DREYFUS.** Learning numbers from graphs. *Applied Statistical Modeling and Data Analysis,* 2005